(19) Europäisches Patentamt / European Patent Office / Office européen des brevets

(11) **EP 3 853 242 B1**

(12) **EUROPEAN PATENT SPECIFICATION**

(45) Date of publication and mention
of the grant of the patent:
**15.05.2024 Bulletin 2024/20**

(21) Application number: **19863709.2**

(22) Date of filing: **19.09.2019**

(51) International Patent Classification (IPC):
**C07K 1/36** (2006.01)   **C07K 14/00** (2006.01)
**C07K 14/435** (2006.01)   **C07K 14/47** (2006.01)
**C12N 9/88** (2006.01)   **C12N 11/02** (2006.01)

(52) Cooperative Patent Classification (CPC):
**C12N 9/22; C07K 14/4702; G01N 33/502;**
**G01N 33/582; G16B 40/10;** C07K 2319/00;
C07K 2319/60; G16B 40/20

(86) International application number:
**PCT/US2019/051827**

(87) International publication number:
**WO 2020/061251 (26.03.2020 Gazette 2020/13)**

(54) **HIGH THROUGHPUT METHOD AND SYSTEM FOR MAPPING INTRACELLULAR PHASE DIAGRAMS**

HOCHDURCHSATZVERFAHREN UND SYSTEM ZUR ABBILDUNG INTRAZELLULÄRER PHASENDIAGRAMME

PROCÉDÉ ET SYSTÈME À HAUT RENDEMENT POUR LA CARTOGRAPHIE DE DIAGRAMMES DE PHASE INTRACELLULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2018 US 201862734063 P**

(43) Date of publication of application:
**28.07.2021 Bulletin 2021/30**

(73) Proprietor: **The Trustees Of Princeton University Princeton NJ 08544 (US)**

(72) Inventors:
• **BRANGWYNNE, Cliff**
**Hopewell, NJ 08525 (US)**
• **BRACHA, Dan**
**Princeton, NJ 08540 (US)**
• **JUMPER, Chanelle**
**Princeton, NJ 08544 (US)**
• **ACKERMAN, Paul**
**Princeton, NJ 08544 (US)**

(74) Representative: **Brand Murray Fuller LLP**
**50 Eastcastle Street**
**London W1W 8EA (GB)**

(56) References cited:
WO-A1-2017/216123   WO-A1-2018/165293
WO-A2-2019/147611   US-A1- 2011 255 753
US-A1- 2016 083 786   US-A1- 2017 219 596
US-A1- 2017 355 977   US-A1- 2018 251 497

• YUAN XUE ET AL: "Live-Cell Imaging of Chromatin Condensation Dynamics by CRISPR", ISCIENCE, vol. 4, 29 June 2018 (2018-06-29), pages 216-235, XP055700100, US ISSN: 2589-0042, DOI: 10.1016/j.isci.2018.06.001
• SHIN et al.: "Spatiotemporal Control of Intracellular Phase Transitions Using Light-Activated optoDroplets", Cell, vol. 168, no. 1-2, 29 December 2016 (2016-12-29), pages 159-171, XP029882177, ISSN: 0092-8674, DOI: 10.1016/j.cell.2016.11.054
• BRANGWYNNE, C.: "Soft Active Aggregates: Mechanics, Dynamics and Self-assembly of Liquid-Like Intracellular Protein Bodies", Soft Matter, vol. 7, 21 February 2011 (2011-02-21), pages 3052-3059, XP055694842, ISSN: 1744-683X, DOI: 10.1039/c0sm00981d

- **BRACHA et al.: "Mapping Local and Global Liquid-Liquid Phase Behavior in Living Cells Using Light-Activated Multivalent Seeds", bioRxiv, 16 March 2018 (2018-03-16), pages 1-16, XP055694847, DOI: 10.1101/283655**
- **SHIN et al.: "Liquid Nuclear Condensates Mechanically Sense and Restructure the Genome", Cell, vol. 175, no. 6, 29 November 2018 (2018-11-29), pages 1481-1491, XP085544101, ISSN: 0092-8674, DOI: 10.1016/j.cell.2018.10.057**
- **WEI et al.: "Nucleated Transcriptional Condensates Amplify Gene Expression", BioRxiv, 21 August 2019 (2019-08-21), pages 1-20, XP055694863, DOI: 10.1101/737387**

Remarks:
The complete document including Reference Table(s) and the Sequence Listing(s) can be downloaded from the EPO website

**Description**

**CROSS-REFERENCE TO RELATED APPLICATIONS**

This application claims the benefit of U.S. Provisional Application No. 62/734,063, filed September 20, 2018.

**TECHNICAL FIELD**

This relates generally to the induced clustering of proteins, and more particularly, to high throughput *in vitro* methods for mapping or screening intracellular interactions by inducing phase separation via, e.g., optically controlling the clustering of proteins.

**SEQUENCE LISTING**

A Sequence Listing is filed electronically herewith .

**BACKGROUND OF THE INVENTION**

Liquid-liquid phase separation (LLPS) is a fundamental mechanism for organizing the contents of cells. LLPS is now recognized as important for driving assembly of a wide range of membrane-less condensates, including cytoplasmic structures such as germ (P) granules, stress granules, miRISC assemblies, and synaptic scaffolds. LLPS also appears to underlie nuclear body biogenesis, including nucleoli, and likely many others. Associated liquid-to-solid phase transitions are also implicated in various diseases of pathological protein aggregation. Intracellular phase transitions arise from weak, multivalent interactions, often mediated by intrinsically disordered proteins/regions (IDPs/IDRs), which are closely related to low complexity sequences and prion-like domains.

Therefore, a method for identifying agents which impact phase separation within cells is desirable, while phase separation itself could be exploited as a readout for underlying biomolecular modifications and agents that modulate them.

**BRIEF SUMMARY**

According to a first aspect of the invention is a high-throughput method for mapping or screening intracellular interactions in vitro comprising the steps of: (a) providing a plurality of cells, each cell expressing a phase separation or aggregation system capable of being controlled by at least one wavelength of light, the phase separation or aggregation system comprising (i) a target protein and (ii) a fluorescent protein (e.g., GFP, mCherry, etc.) or an attached fluorophore; (b) placing at least one of the plurality of cells in a well at a first temperature; (c) introducing at least one chemical or biological agent to the well at a first concentration; (d) irradiating the well with the at least one wavelength of light, in a constant or pulsed fashion, and allowing the phase separation or aggregation system to form condensates; (e) irradiating the at least one of the plurality of cells with an additional wavelength of light to cause the fluorescent protein or an attached fluorophore to fluoresce; (f) quantifying phase separation or aggregation based on an amount of fluorescence within a first region and a second region of the plurality of cells, the first region containing a condensate and the second region not containing a condensate, to map or screen intracellular interactions. The target protein is the protein in the phase separation or aggregation system for which agents that modulate its phase separation behavior would be identified through the disclosed screening approach, and is generally a protein capable of driving phase separation, either through self-interactions (e.g., FUS-FUS interactions), or a network of heterotypic (non-self) interactions (e.g., G3BP-Caprin interactions). These phase separation or aggregation systems, when activated, cause a target molecule or class of molecules to phase separate or aggregate. For example, intrinsically disordered regions from a nuclear protein such as BRD4, FUS, and TAF15 phase separate into liquid condensates that preferentially form in low-density euchromatic regions, and mechanically exclude chromatin as they grow.

In some embodiments, the phase separation or aggregation system may be an optoDroplet system (see US Pub. No. 2017/0355977), a CasDrop system (see PCT/US2019/014666), a Corelet system (see US Pub. No. 2018/0251497), or a PixELL system (see Dine et al.., "Protein Phase Separation Provides Long-Term Memory of Transient Spatial Stimuli", Cell Systems 6, 655-663 June 27, 2018).

In some embodiments, for example utilizing variants of the CasDrop system, the cell is configured to express a phase separation or aggregation system comprising two constructs: (i) a first construct comprising enzymatically dead Cas9 fused or attached to two or more repeating sequences, each repeating sequence including a receptor protein sensitive to at least one wavelength of light, and (ii) a second construct comprising a cognate partner of the light-sensitive receptor protein fused to at least one fluorescent protein and at least one gene regulatory protein having a full length or truncated low complexity or intrinsically-disordered protein region, or other folded proteins known to promote self-interactions. The target protein may be one or more of these gene regulatory proteins, for which agents that modulate its phase separation behavior would be identified through the disclosed screening approach.

At least one of the cells is then placed in a well. At some point thereafter, two steps may occur in any order: a chemical or biological agent can be placed in the well, and the well can be irradiated with the at least one wavelength of light that controls the phase separation or aggregation system to bind to a target molecule and form condensates. Optionally, the chemical or biological agent is a compound from a small molecule library. Optionally, the chemical or biological agent is a component of a genetic knockout or knockdown screening system, such as TALEN, shRNA, siRNA and CRISPR-KO.

Optionally, after the agents have been placed in the wells, and the cells have been irradiated, cells may optionally be fixed and possibly stained using standard methods known to those of skill in the art (*e.g.,* immunohistochemistry), to render their internal structures immobilized for later analysis. Then, the living or fixed cells can be irradiated with a different wavelength in order to allow the fluorescent protein and/or other fluorophores to fluoresce. Fluorescence can be detected in a region containing a condensate, as well as in a region that does not contain a condensate. Optionally, this involves capturing an image of the at least one of the plurality of cells while the fluorescent protein is fluorescing.

The phase separation or aggregation can be quantified based on an amount of fluorescence detected within the two regions. The amount of fluorescence reflects the concentrations of the target molecules in the two regions; for example, fluorescence correlation spectroscopy can be used to develop a calibration curve to allow one of skill in the art to translate between intensity and concentration. Optionally, the method also includes determining a first concentration based on the amount of fluorescence in the region with a condensate and determining a second concentration based on the amount of fluorescence in the region without a condensate.

Optionally, the method includes utilizing image processing software to identify one or more potential first regions and one or more potential second regions. Optionally, the method includes measuring fluorescence within at least one of the potential first and second regions. Optionally, the method includes determining a concentration within a region based on the measured fluorescence within the region. Optionally, when an image of the fluorescence is captured, the image is sent to a machine learning algorithm, preferably one trained to estimate concentrations based on the characteristics of the image. Optionally, quantifying phase separation comprises determining a threshold in changes in image parameters that allow determination of percentage of cells in a given well that phase separate. Optionally, toxicity associated with assay conditions is determined by examining a metric comprised of the number of viable cells detected within each well, by image analysis algorithms.

Optionally, the method includes repeating some of the steps under different light and/or temperature conditions. The repeated steps may include irradiating the wells with constant or pulsed sequences of light to form or dissolve condensates, irradiating the cells to cause the fluorescent protein to fluoresce, and detecting the fluorescence in different regions.

Optionally, the method may include generating some or all of a phase diagram (for example binodal and/or spinodal phase boundaries) utilizing the quantified phase separation or aggregation.

Optionally, the method includes comparing the quantified phase separation or aggregation to a baseline quantified phase separation or aggregation, and optionally signaling to a user when the difference exceeds a predetermined threshold.

Also disclosed herein but not claimed is a system for high throughput mapping or screening of intracellular interactions. The system includes one or more light sources capable of emitting at least two wavelengths - one wavelength that a receptor protein within a cell is sensitive to, and one wavelength of light a fluorescent protein within the cell is capable of absorbing. The system also includes a detector for capturing an image of a plurality of fluorescent proteins within the cell. The system also includes memory and one or more processors. The memory includes instructions to be executed by the processor(s), resulting in one or more processor containing instructions that, when executed by at least one processor: the one or more light sources irradiating a well with the at least one wavelength of light the receptor protein within the cell is sensitive to, as well as irradiating the at least one well with the at least one wavelength of light the fluorescent proteins are capable of absorbing, receiving an image from the detector, detecting a first region in the image with a first intensity, and a second region of the image with a second intensity, where the first intensity being higher than the second intensity, and then determining a concentration of the first region based on the first intensity and a concentration of the second region based on the second intensity.

Also disclosed herein, but not claimed, is an alternative high-throughput method for mapping or screening intracellular interactions. The method involves providing cells configured to express a non-light activated phase separation or aggregation system (*e.g.,* a system configured to overexpress phase-separation prone proteins, such as gene regulatory proteins having a full length or truncated low complexity or intrinsically-disordered protein region (*e.g.,* FUS-FUS interactions, or hnRNPA1-hnRNPA1 interactions), or other folded proteins known to promote self-interactions (*e.g.,* G3BP-G3BP interactions), or a network of heterotypic (non-self) interactions (*e.g.,* G3BP-Caprin interactions), or phase separation, where the phase separation or aggregation system comprises a target protein, or a target protein and a fluorescent protein. These phase separation or aggregation systems, when expressed, will naturally result in causing a target molecule or class of molecules to phase separate or aggregate. The method otherwise is similar to the previously disclosed method, except there is no requirement to irradiate (or repeatedly irradiate) the cells, since these phase separation or aggregation systems are not activated by light.

## BRIEF DESCRIPTION OF THE DRAWINGS

Figure 1 is a flowchart depicting one embodiment of a disclosed method.

Figure 2A is an example of a binodal phase diagram produced as part of the disclosed method, for FUS using the Corelet system, generated using automated image analysis methods.

Figure 2B shows example data from a 100-drug screen using the disclosed methodology, showing the percentage of phase separated cells as a function of the number of cells analyzed. Open circles represent the positive control (10 wells), filled circles are results from test wells, and hits with a box around them indicate a high confidence hit.

Figure 3 is a block diagram of one embodiment of a disclosed system.

Figure 4A is an image illustrating that when a phase separation or aggregation system of CasDrop system (comprising dCas9 fused to SunTag, scFv fused to sfGFP and iLID, and a transcriptional regulator (BRD4ΔN) fused to mCh and sspB), is co-expressed with sgRNA for telomeres, bright GFP foci (410) are observed to colocalize with telomeric repeat-binding factor TRF1, indicating successful scaffold targeting.

Figures 4B-4D are images illustrating that, upon activation of the phase separation or aggregation system by irradiating it with blue light over time, BRD4ΔN-mCh-sspB liquid droplets nucleate and grow at the seeded telomeres (time t=0 seconds (Fig. 4B), t=11 seconds (Fig. 4C), and t=176 seconds (Fig. 4D) after the beginning of blue light irradiation).

## DETAILED DESCRIPTION

Disclosed is an *in vitro* method for high throughput detection of biomolecular interactions, including very weak interactions, between a target biomolecule and an array of small molecules and/or biologics, which might be used to identify molecules that interfere with these biomolecular interactions, protein phase separation, and/or associated irreversible aggregation. This technique provides the ability to obtain systematic, potentially proteome-wide mapping of phase behavior within cells. The process could be used, for example, in interrogating an array of different protein sequences, which will be useful for developing strategies for the design of engineered proteins with new types of desired phase behavior, for any number of biotech applications. Additionally, because phase separation is indicative of intermolecular interactions, the detection of phase separation might be utilized in a manner similar to a readout for chemicals (drugs) of interest. That is, the drug might not be used to modulate phase separation in patients; the drug might ultimately just be used to perturb the underlying interactions, in a physiological context where phase separation is not even occurring.

This technique also provides for high throughput high sensitivity screening of large libraries of compounds against specific biological molecules in the intracellular or possibly extracellular environment, and/or high throughput drug discovery screening of large libraries of compounds for treating diseases in which phase separation plays a key role. For example, various cancers, including Ewings Sarcoma, are thought to be driven by hyperactive transcription, which may occur through transcriptional condensates, in the latter case driven by EWS-FLI1 protein fusion proteins. Identifying compounds that disrupt this phase behavior could provide an important therapeutic strategy. Phase separation is also thought to play a role in diseases where irreversible protein aggregates or abnormal inclusion bodies are observed. The diseases may include but are not limited to Amyotrophic Lateral Sclerosis (ALS), Alzheimer's disease, Huntington's disease, and Parkinson's disease. The described technology would allow direct observation of the ability of a potential drug to affect the aggregates/condensates assembly dynamics, material properties (*e.g.,* viscoelasticity and morphology), and reversibility, including the potential to dissolve or liquify aggregates, decreasing their density, or changing their composition.

The disclosed invention provides an *in vitro* method for high throughput and high sensitivity screening inside live cell-lines, including human cells. It is envisioned that this technique could also be utilized *in vitro* in systems beyond cell lines, such as within model organisms (including, *e.g.,* nematodes such as *C. elegans,* bacteria such as *E. coli,* and yeasts such as *S. cerevisiae* ). There are key advantages for utilizing the invention in living cells, rather than with purified components. Compared to current drug discovery screening techniques, which are typically conducted in-vitro with purified biological targets, *in vitro* screening in live cells provides at least two distinct advantages. First, screening is conducted under environmental conditions and macromolecular composition (i.e. crowded intracellular milieu) similar to those of the intended ultimate use of the drug, within patients. Moreover, the screened drug will already be subjected to the same chemical modifications, processing, turnover, and competing interactions with other biomolecules, thus allowing superior screening capabilities and significantly narrowing down the number of potential drug candidates without the need of further extensive tests. The unique conditions present in live cells *in vitro* may promote beneficial specific or collective cellular activity that would not occur in artificial, in-vitro solutions, and therefore would not provide a hit under traditional in-vitro screening techniques. And second, it obviates the need for isolating the biological targets being tested for screening assays; instead simple cloning techniques are used to induce expression of the proteins/RNAs being targeted. Moreover, expressing the proteins targeted for screening in human cell-lines ensures having all the required processing steps and post-translational modifications as well as having all the different isoforms, and at the cellular-relevant concentrations.

The disclosed invention also allows sensitive detection of even extremely weak interactions between the target protein and screened compounds. The method allows this because the described technique does not require direct detection of molecular interactions but rather, identifying offsets in mapped phase diagrams in the absence and presence of screened molecules, which may reflect minute changes in the relevant network of biomolecular interactions.

The disclosed method may utilize the expression of photo-induced phase separating protein condensates containing a screening target protein, which could be either (I) a self-interacting protein, including those found in pathological aggregates or (II) any protein of interest fused to a self-interacting polypeptide, such as FUS PLD. Then, phase diagrams of the photo-induced condensates should be reconstructed in the presence or absence of a library of drug candidates, or under an array of genetically different conditions (*i.e.,* a genetic screen) by high throughput imaging and automated analysis over a large population of cells. For example, under small molecular screens, shifts in the mapped phase diagrams would suggest whether a protein of interest of type I interacts with any of the screened molecules under physiological conditions and in the case of proteins of interest of type II, whether any of the screened molecule protein may induce dissolution, liquification, or composition change in pathological aggregates containing condensates.

The synthetic photoactivatable condensates may be constructed within the living cell or organism, by, *e.g.,* expressing various self-interacting proteins as is known in the art. Alternatively, for in vitro studies, the proteins may be expressed and purified using known techniques, including, *e.g., E. coli*-based protein expression systems and gel chromatography.

Phase diagrams for the protein may then be mapped and can be analyzed to extract detailed information about the thermodynamics of underlying biomolecular interactions. The phase diagrams are unique to specific proteins, and a phase diagram is responsive to mutations and post-translational modifications.

Additionally, a neural network or other machine learning approach may be trained using images acquired from a camera attached to a high throughput data acquisition system, using an algorithm or methodology known to those in the art. Once the algorithm is trained, an array of wells, each containing, *e.g.,* the target protein and potentially one compound from a small molecule library, can be used to run through the high-throughput data acquisition system. By controlling the light that is exposed to the wells, images can be collected as light conditions are modified. When those images are run through the neural net, the phase diagrams can be automatically generated and analyzed for each well.

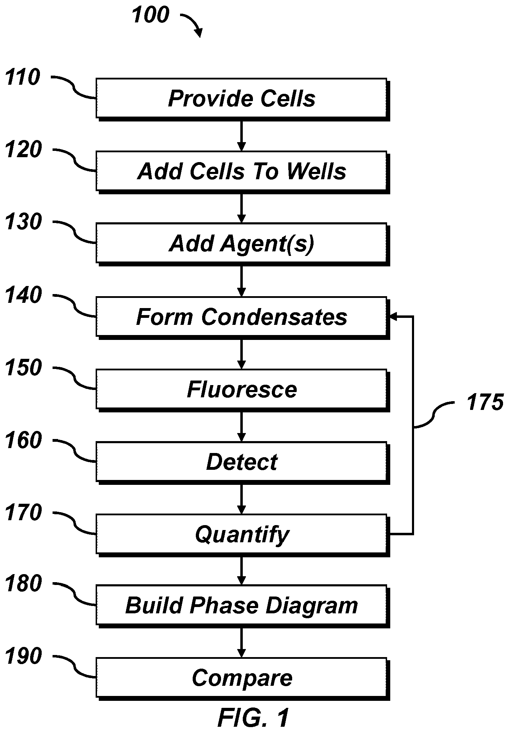

Referring to Fig. 1, one embodiment of the high-throughput method (100) for mapping or screening intracellular interactions initially involves providing a plurality of cells (110) configured to express a phase separation or aggregation system capable of being controlled by at least one wavelength of light. The cells may be any cell type, but is preferably a cell of a mammal, and more preferably a human cell. As one of skill in the art will recognize, various model organisms (*e.g.,* nematodes, bacteria, yeast, etc.) may be used instead of, *e.g.,* human cells.

Phase separation or aggregation systems are generally known to those of skill in the art. The phase separation or aggregation system may also contain a fluorescent protein.

As is known to those of skill in the art, the phase separation or aggregation systems generally include one or more proteins and/or nucleic acids that work together to provide various functions, including (1) the ability to target, *e.g.,* a particular protein, molecule or gene, and (2) the ability to phase separate. For optically controlled phase separation and aggregation systems, the proteins and/or nucleic acids also work together to provide a third function: the ability to control the phase separation using one or more wavelengths of light.

In some embodiments, the phase separation or aggregation system is an optoDroplet system (see US Patent Pub. No. 2017/0355977). Briefly, an optoDroplet system comprises two segments fused together, where the first segment is a light-sensitive protein (such as Cry2, Cry2olig, PhyB, PIF, light-oxygen-voltage sensing (LOV) domains, or Dronpa) that is sensitive to at least one wavelength of light, and the second segment could be a low complexity sequence (LCS) or an intrinsically disordered protein region (IDR) (such as those present in FUS, Ddx4, and hnRNPA1), or in some cases a folded protein/domain. The optoDroplet system is produced using known techniques. The optoDroplet system functions by irradiating the optoDroplet system with a wavelength of light the light-sensitive protein is sensitive to. The optoDroplets then self-assemble while exposed to that light, and the assembly of the LCSs or IDRs can cause phase separation (or droplet formation) to occur. When the light is turned off, this phase separation can be reversed.

In some embodiments, the phase separation or aggregation system is a Corelet system (see US Patent Pub. No. 2018/0251497). Briefly, the Corelet system comprises two constructs. The first construct is a light-sensitive protein that is sensitive to at least one wavelength of light fused to a self-assembling protein subunit (such as ferritin). The second construct includes a cognate partner of the light-sensitive protein fused to an LCS or IDR, or in some cases a folded protein/domain. The Corelet system is produced using known techniques. The Corelet system functions by first allowing the self-assembling protein subunits to self-assemble into a "core", where the core has light-sensitive proteins facing outwards. The second construct is simply present nearby. When exposed to light, the light-sensitive protein activates, and seeks to bind with its cognate partner on the second construct, resulting in a central "core" that is surrounded by the LCSs or IDRs of the second constructs. Then, like the optoDroplet system, the assembly of those LCSs or IDRs, or possibly a folded protein/domain, can cause phase separation to occur.

In some embodiments, the phase separation or aggregation system is a PixELL system (see Dine et al.., "Protein Phase Separation Provides Long-Term Memory of Transient Spatial Stimuli", Cell Systems 6, 655-663 June 27, 2018). Briefly, a PixELL system is a subset of the Corelet concept and generally comprises a system with two components. Each component comprises an LCS or IDR fused to either PixD or PixE (from Synechocystis sp. PCC6803). PixD and PixE associate in the dark into large multi-subunit complexes (thought to exhibit 10:4 or 10:5 PixD:PixE stoichiometry) that dissociate into dimers of PixD and monomers of PixE within seconds upon blue light stimulation. By fusing the LCSs or IDRs to PixD and PixE, the formation of the complexes in the dark leads to large numbers of LCSs or IDRs in close proximity, which, as mentioned above, can cause phase separation to occur. PixEll systems can be produced using known techniques.

In some embodiments, the phase separation or aggregation system is a CasDrop system (see PCT/US2019/014666). The CasDrop system, as the name implies, makes use of a Cas-based genomic targeting protein to target where phase separation (i.e., droplet formation) will occur. The CasDrop system often includes two components - a first component comprising a Cas-based genomic targeting protein fused to a first sequence, where the first sequence includes a light-sensitive receptor, a chemical-sensitive receptor, a light-sensitive oligomerization protein, and/or a non-light sensitive dimerization module. The second component of CasDrop is an LCS or IDR fused to a cognate partner (or complementary dimerization domain) to whatever is used in the first sequence. CasDrop can be produced using known techniques. CasDrop works by first providing a specific genomic target loci, complementary single guide RNA, and the CasDrop system in a cell, and then exposing the CasDrop system to a light or chemical that causes the first sequence in the first component to bind to the cognate partner of the second component, at a location around the specific genomic target loci. Again, similar to optoDroplets and Corelets, the system would then have the LCSs or IDRs, and possibly a folded protein/domain, of the second component assembled together, which can cause phase separation / droplet formation.

In some embodiments, a particular implementation of a CasDrop system can be utilized, where the phase separation or aggregation system is capable of expressing two constructs: (i) a first construct comprising enzymatically dead Cas9 fused or attached to two or more repeating sequences, each repeating sequence including a receptor protein sensitive to at least one wavelength of light, and (ii) a second construct comprising a cognate partner of the light-sensitive receptor protein fused to at least one fluorescent protein and at least one gene regulatory protein having a full length or truncated low complexity or intrinsically-disordered protein region, or other folded proteins known to promote self-interactions, a network of heterotypic (non-self) interactions, or phase separation.

Referring again to Fig. 1, the cells are then placed into a well (120). In some embodiments, cells are placed into each well in a well array (such as a 384-well array). The well is typically maintained at a predetermined temperature using equipment known to those of skill in the art.

Disclosed herein, but not claimed, are some instances where no light is required to induce phase separation/aggregation. Instead of expressing light-sensitive phase separation or aggregation system, the cells are configured to express phase separation-prone proteins at concentrations sufficient to induce their phase separation and/or aggregation, possibly as a function of altered conditions including changes in temperature or solution osmolarity.

The next two steps may be performed in any order, depending on the needs of user. In some embodiments, at least one chemical or biological agent is introduced to the well (130) at a first concentration, after which the well is irradiated with a wavelength of light that can control the phase separation or aggregation system, allowing the phase separation or aggregation system to form condensates (140). Those steps may be reversed.

In some embodiments, the chemical or biological agents may include a compound from a small molecule library. In some embodiments, the chemical or biological agents may include a component of a genetic knockout or knockdown screening system (e.g., TALEN, shRNA, siRNA and CRISPR-KO).

The system and method may be highly automated in a manner known to those of skill in the art. For example, cells, growth media, and the chemical or biological agents may be distributed to a large number of wells (e.g., multiple 384 well plates with a multiplicity of plates such that the total number of wells may be in excess of 10,000) using commercially available robotic automation systems.

Referring again to Fig. 1, the method continues by irradiating the cells with at least one wavelength that causes the fluorescent protein or fluorophore to fluoresce (150). This wavelength of light could be different from the wavelength of light that controls the phase separation or aggregation system.

In some embodiments, cells are then fixed and stained using an appropriate technique known to those of skill in the art, which may involve introducing/binding one or more fluorophors to the phase separation or aggregation system as appropriate. This procedure may also be performed in an automated fashion, for example by using commercially available robotic automation systems to distribute fixative and buffers to the wells. For example, acetone fixation can be used, where cells can be fixed in -20 °C acetone for 5-10 minutes. Alternatively, cells can be fixed and permeabilized in a three-step process: (1) fixing in 3-4% paraformaldehyde for 10-20 minutes, (2) rinsing briefly with phosphate buffered saline (PBS), and (3) permeabilizing with 0.5% Triton X-100 detergent solution for 10 minutes. Similarly, the staining process (which follows the fixation and permabilizing steps), can be accomplished with appropriate fluorescent dyes and/or antibody conjugates known to those of skill in the art. Two examples include the use of 4',6-diamidino-2-phenylindole (DAPI) or Alexa Fluor 488 dye.

The next step involves measuring (160) the fluorescence. This may be done using any technique known to those of skill in the art. In one embodiment, an image of the at least one of the plurality of cells is captured while the fluorescent protein is fluorescing. In some embodiments, one or more processors may cause components of a microscope to change the position of wells such that different wells come into focus.

In some embodiments, additional processing is done using the image to quantify the phase separation and aggregation (170). In some embodiments, image processing software is used to measure the intensity of the fluorescence at various locations in the captured image, and the intensities or related image analytic metrics can be used to quantify phase separation and aggregation. For example, the standard deviation of pixel intensity can be used to quantify the phase separation and aggregation, since standard deviation should increase significantly upon phase separation/aggregation.

In some embodiments, quantifying phase separation can include determining a threshold in changes in image parameters that allow determination of percentage of cells in a given well that phase separate. For example, in some embodiments the maximum pixel values are compared with the mean, where the max could be, *e.g.,* the top 5% brightest pixels, such that when max/mean plus an offset exceeds a threshold as defined below, the cell is identified as phase separating. The threshold and offset may be determined by a non-phase separating control such that, e.g., 95% of the control is deemed to be not phase separating.

In some embodiments, toxicity associated with assay conditions is determined by examining a metric comprised of the number of viable cells detected within each well, using known image analysis algorithms. Toxicity could be quantified as the density of detected cells divided by the seeded density (or density in untreated control wells). Image analysis could also detect changes in morphology associated with toxicity, for example by quantifying the ellipticity of cell nuclei, or calculating convex hull to quantify roughness of the nuclear envelope.

In some embodiments, a separate experiment is performed to generate a calibration curve to convert from fluorescence intensity to concentration, for example using concentration standard biomolecule solutions and possibly utilizing fluorescence correlation spectroscopy (FCS).

In some embodiments, image processing software uses known techniques to identify a region where condensates exist and a region where condensates do not exist, then measures the intensity of the fluorescence in only those two regions.

In some embodiments, the image is sent to a machine learning algorithm, trained using techniques known to those of skill in the art to quantify phase separation or aggregation based on the sent image.

In some embodiments, some steps are repeated (175) under different light and/or temperature conditions. The repeated steps may include irradiating the wells with pulses of light to form condensates, irradiating the cells to cause the fluorescent protein to fluoresce, and detecting the fluorescence in different regions.

In some embodiments, the structures that can be induced start out as liquids but can transition into pathological and irreversible aggregates over time. In those instances, this technique can be used to monitor a time-dependent transition into an irreversible aggregation state by changing the time that the cells are subject to constant or pulsed activating light. In some embodiments, that is done by having different wells experience different exposures. In some embodiments, the wells are exposed to different light conditions, and the intensity of fluorescence at the start of each time period is monitored, as a fully reversible aggregation would result in the standard deviation of the starting intensity being substantially the same each time, while with irreversible aggregation one would see changes in intensity as more and more of the aggregation was no longer reversible.

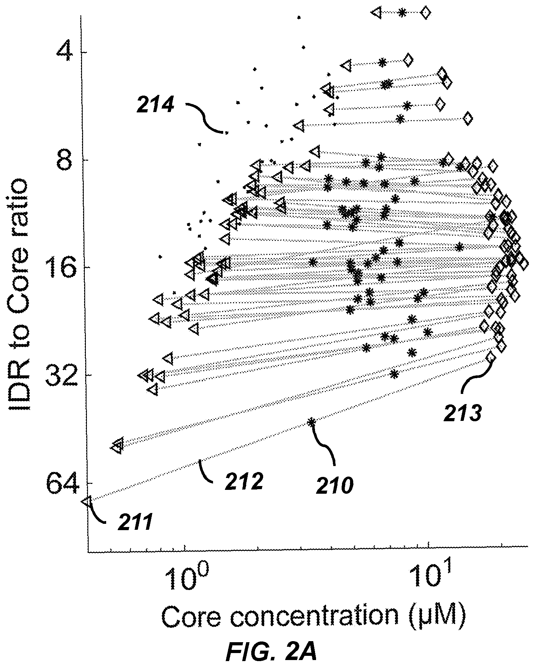

The method may also comprise generating a phase diagram (180) based on the quantified phase separation or aggregation. As will be understood by those of skill in the art, multiple test conditions will be needed to generate a phase diagram. In some embodiments, between 10 and 100 test conditions are used to develop a phase diagram. An example phase diagram generated using the disclosed method is shown in Fig. 2A. Figure 2A is a phase diagram of FUSn Corelets, plotted as Core concentration versus IDR to Core ratio. Asterisks (210) represent the initial average nuclear value of cells that phase separate while small solid circles (214) represent cells that do not phase separate. Open triangles (211) connected by a grey line (212) to open diamonds (213) represent the average values of the dilute (open triangles, 211) and dense (open diamonds, 213) phases of cells demonstrating phase separation. Note that in some embodiments, qualitatively similar phase diagrams may be generated, but where the x-axis denotes changes in other system parameters, for example temperature or ionic strength.

For Fig. 2A was produced as follows. Automated nuclear segmentation was achieved using initial core distribution excluding nucleolus and lamin. Cells exhibiting phase separation have a distinct bimodal intensity distribution. Limits due to image resolution were accounted for through filtering and erosion of segmented binary masks. Dense and dilute phase masks and mean pixel intensity were then determined for illuminated cells. Finally, FCS calibration of intensity to concentration was applied.

Referring again to Fig. 1, the method may comprise comparing (190) the quantified phase separation or aggregation to a baseline phase separation or aggregation. In some embodiments, the baseline phase separation or aggregation may be the phase separation or aggregation quantified for another well, such as a well in the same well array but where no chemical or biological agent was added, or where a positive or negative control agent was added. In other embodiments, it may be the phase separation or aggregation quantified for a previous iteration of the same well, at a different temperature or light condition.

In some embodiments, if the comparison results in a difference between the quantified phase separation or aggregation and the baseline phase separation or aggregation that exceeds a predetermined threshold, then a signal is sent to a user or a remote device indicating, *e.g.,* the agents and conditions for which the threshold was exceeded.

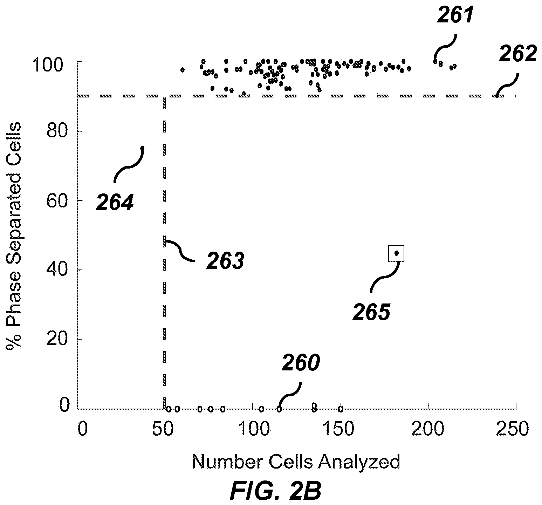

In this manner, the disclosed method can aid in, *e.g.,* drug development by rapidly narrowing the potential agents and conditions which can impact biomolecular interactions within living cells, which under specific conditions may lead to phase separation. Referring to Fig. 2B, example data from a 100-drug screen is shown, showing the percentage of phase separated cells as a function of the number of cells analyzed. Open circles (260) represent the 10 wells used for positive control, which were designed using an agent, hexanediol, known to fully inhibit phase separation. Filled circles (261) are results from test wells. Horizontal dashed lines (262) indicate the hit "cut-off", which for this example was defined as three standard deviations from the mean of the 10 wells used for negative control. Vertical dashed lines (263) indicate the cell count "cut-off", which for this example was defined as the lower limit of number of cells analyzed in the positive control. Cells below the horizontal cut-off represent a hit (264, 265). Hits (264) to the left of the vertical cut-off (263) indicate potential cell toxicity and should be evaluated manually and potentially re-screened at lower concentration. The hit with the box around it (265) represents a high confidence hit (>3 SDs from mean of negative control and sufficient number of cells analyzed).

In some embodiments, the method also includes identifying false-positive hits. For example, instead of the desired hits that targeted the IDR-IDR interactions (or folded protein-folded protein interactions that could similarly drive phase separation), might target, *e.g.,* the light-activation interactions (*e.g.,* an iLID-sspb interaction). These would constitute a false positive, as they inhibit phase separation by disrupting interactions intrinsic to the functioning of the platform that had been designed to facilitate phase separation. These false positives can be identified by doing simultaneous or secondary screens with a complementary optogenetic system. For example, if a first screen utilized iLID-sspb attached to TDP43, then a secondary screen using a complementary system, such as Cry2 attached to TDP43, could be used. In this example, if one detects perturbations in only one of the two screens, it would indicate the perturbation was likely due to optogenetic disruption, while if perturbations were detected in both screens, it would indicate true positive targeting of the desired TDP43 interactions.

While there are advantages to utilizing the disclosed technique with living cells *in vitro,* it is also disclosed herein, but not claimed, that the above-disclosed method could also be accomplished "in vitro," utilizing purified proteins, which may include either a light-activated or non-light activated protein that comprise a given phase separation/aggregation system as disclosed above. When considering a high-throughput method for mapping or screening biomolecular interactions in-vitro, using purified proteins, the method follows a very similar path as the method described previously. The method begins by providing a plurality of purified proteins, which include a phase separation or aggregation system comprising a target protein or a target protein and a fluorescent protein. The purified proteins in at least one well at a first temperature, after which at least one chemical or biological agent is introduced to one or more of the wells at a first concentration. The phase separation or aggregation system is then allowed to form condensates under appropriate conditions, and then the wells are irradiated with a wavelength of light that causes the fluorescent protein or an attached fluorophore to fluoresce. Finally, the phase separation or aggregation is quantified based on an amount of fluorescence within a first region and a second region of a given well, the first region containing a condensate and the second region not containing a condensate.

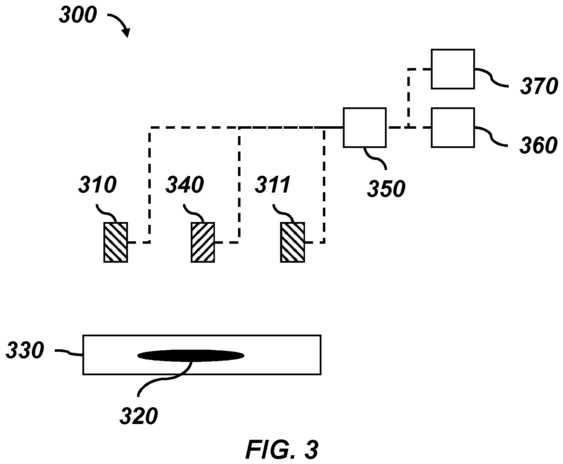

Referring to Fig. 3, an embodiment of a system for high throughput mapping or screening of intracellular interactions is disclosed. The system (300) includes one or more light sources (310, 311) configured to irradiate a cell (320) within a well (330) with at least one wavelength of light that a receptor protein within the cell is responsive to, as well as irradiate the cell (320) with at least one wavelength of light that a plurality of fluorescent proteins or other fluorophores within the cell are capable of absorbing, causing them to fluoresce.

The system (300) also includes at least one detector (340) configured to capture an image of the plurality of fluorescent proteins within the cell while the fluorescent proteins or other fluorophores are fluorescing.

The system (300) also includes memory (360) containing instructions for one or more processors (350). The instructions cause the one or more processors to perform several tasks. First, they cause at least one light source (310) to irradiate the at least one well with the at least one wavelength of light the receptor protein within the cell (320) is sensitive to. They later cause at least one light source (311) to irradiate the at least one well with the at least one wavelength of light the fluorescent proteins/fluorophores within the cell (320) are capable of absorbing, sufficient to cause the proteins/flourophores to fluoresce. The instructions also cause the processor (350) to receive an image from the detector, where the image comprises a plurality of actual or effective pixels, each pixel having an intensity. Note that, for example, in scanning confocal microscopy, an image is created from an array of detection events as the single laser focus is scanned across the cells, so the image that results may be thought of as comprising multiple "effective" pixels, although the detector itself does not capture an image with multiple actual pixels. In some embodiments, the detector (340) does not collect light until the processor (350) sends a signal indicating a picture should be taken, while in some other embodiments, the detector (340) continuously collects data and sends images to the processor, but the processor only considers an image received at an appropriate time (*e.g.,* the first image received 5-10 milliseconds after the light source (311) irradiates the well with an appropriate wavelength to cause the fluorescent proteins to fluoresce). In some embodiments, the image is taken a fixed period of time after the start of the irradiation causing the proteins to fluoresce.

The instructions also cause the processor to quantify phase separation or aggregation based on the plurality of pixel intensities.

In some embodiments, the system (300) also includes one or more other components (370), such as a display, a wired or wireless communications interface, an automated stage capable of finding and moving to and bringing into focus any particular well, etc.

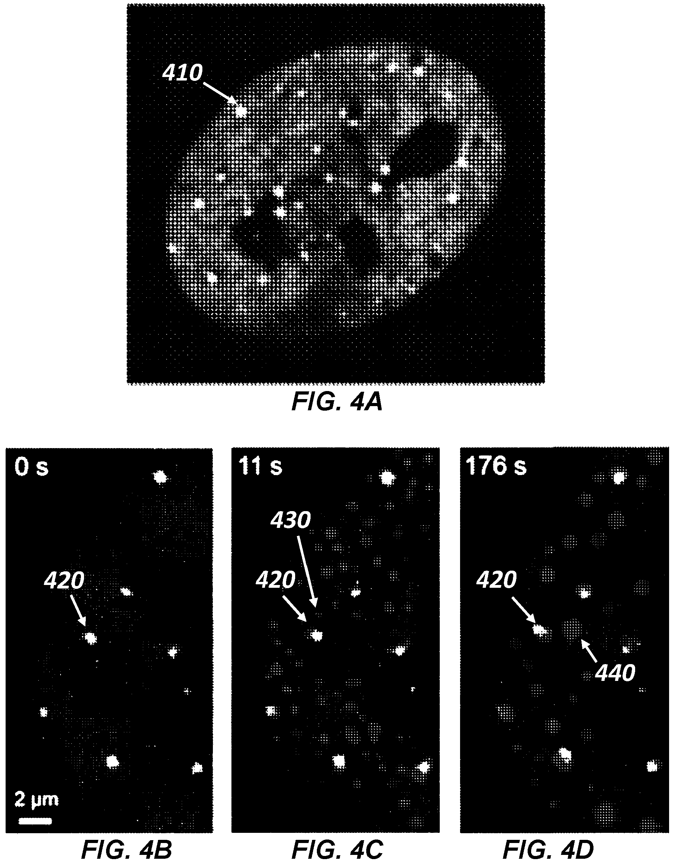

Below is an example of how the phase separation and aggregation system can cause droplets to coalesce. Referring to Fig. 4A, when a CasDrop system (comprising dCas9 fused to SunTag, scFv fused to sfGFP and-iLID, and a transcriptional regulator (here, BRD4ΔN) fused to mCh and sspB), is co-expressed with sgRNA for telomeres, bright GFP foci (410) are observed to colocalize with telomeric repeat-binding factor TRF1, indicating successful scaffold targeting. Referring to Figs. 4B-4D, upon activation of the phase separation or aggregation system by irradiating it with blue light, BRD4ΔN-mCh-sspB liquid droplets nucleate and grow at the seeded telomeres. As seen in Fig. 4B (time t = 0 seconds after irradiating with blue light), there are a number of bright red areas already formed (420). The image has been modified such that one cannot see the diffuse red coloration appearing in the majority of the cell. However, at t=11 seconds of blue light irradiation (Fig. 4C), one can see areas where, rather than a diffuse red background, there are numerous small droplets, each appearing as a slightly brighter red area (430). Some of these slightly brighter areas can be seen around the original bright red areas (420). At t=176 seconds (Fig. 4D), it can be seen that some of the droplets have combined, as there are far fewer of the slightly brighter areas, but those areas are far larger (440), as the droplets continue to grow and interact with each other. Interestingly, the number of droplets that are nucleated away from telomeres depends sensitively on the activation protocol: for a rapid increase in blue light, many droplets nucleate throughout the nucleoplasm, while for a light intensity ramping protocol, droplets nucleate almost exclusively at the telomere loci. This behavior is consistent with the classic physics of nucleation barriers for phase separation: for rapid activation, the system becomes deeply supersaturated in a short time, lowering the nucleation barrier to form many nuclei. For the incremental activation, on the other hand, initially the supersaturation level is low, allowing for preferential condensation at the seeded sites and then in later stages, existing droplets keep growing rather than forming costly new nuclei.

Simulations of one very specific instantiation within the confines of the disclosed techniques confirm this picture. To simulate, a minimal model of mechanical exclusion of chromatin by droplet formation is needed.

The following relation between pressure and size for a spherical cavity can be considered, derived from a neo-Hookean strain energy relation to determine the inward pressure on a cavity with deformation ratio $\lambda$ in an incompressible elastic medium of Young's modulus $G$:

$$\frac{P}{G} = \frac{5}{6} - \frac{2}{3\lambda} - \frac{1}{6\lambda^4} \qquad\qquad (\text{Eq. 1})$$

This pressure, which reflects the energetic cost of deforming the chromatin, complements classical nucleation theory to describe the energetics of droplet formation in a dense elastic matrix. We add the energy cost of deforming the elastic chromatin matrix to the contributions of bulk chemical potential gain and surface tension cost to obtain the total free energy cost to create a spherical droplet of radius $R$, obtaining:

$$\Delta F(R) = 4\pi R^2 \gamma - \frac{4}{3}\pi R^3 \left( \Delta\mu \cdot c_{drop} - \frac{5}{6}G - \frac{2}{3\lambda} - \frac{1}{6\lambda^4} \right) \qquad (\text{Eq. 2})$$

where $\gamma$ is the surface tension of the droplet, $\Delta\mu$ is the chemical potential difference between molecules in the supersaturated solution and the droplet phase, $c_{drop}$ is the saturated bulk concentration of molecules inside the droplet, $\lambda =$

$R/r_{mesh}$, and $r_{mesh}$ is a typical local mesh size in the chromatin network. Since $r_{mesh}$ << $R$, the deformation ratio $\lambda$ is very large (see Estimation of parameters), and we obtain the following simplified result:

$$\Delta F(R) = 4\pi R^2 \gamma - \frac{4}{3}\pi R^3 \left(\Delta\mu \cdot c_{drop} - \frac{5}{6}G\right) \qquad\qquad \text{(Eq. 3)}$$

For values of $\Delta\mu \cdot c_{drop}$ less than the critical pressure $P_c$ = 5G/6, the free energy increases to infinity for large $R$, suggesting that for sufficiently dense chromatin, droplet size is restricted. However, when $\Delta\mu c_{drop} > Pc$, the droplet can grow without constraint. If the elastic environment is heterogeneous, as chromatin density is in the cell, then regions of low stiffness should be favored for phase separation and out-compete denser regions for molecules.

One can estimate the key parameters in this model based on literature:

$\gamma$ = 4 x $10^{-7}$ N/m for nucleolar protein in vitro.

$r_{mesh} \approx$ 7-13 nm. A rough estimate of pore size can be given by the mean free path between chromatin fibers

$$r_{mesh} = r_c^3 \sqrt{\frac{1-f}{f}}$$

based on the chromatin size and volume fraction: where $r_c$ is the width of a chromatin fiber, estimated at 7 nm and $f$ is the volume fraction of chromatin. The fraction has been estimated by electron microscopy at approximately .12-.21 in euchromatin and about .37-.52 for heterochromatin, giving pore sizes of about 14 nm and 7 nm respectively.

$c_{drop}$ has been estimated using fluorescence correlation spectroscopy both in the similar biomimetic Corelet system and for in vitro droplets to be approximately 5-10 x $10^{-5}$ molecules/nm3.

$\Delta\mu$ is estimated at approximately 2-5$k_B T$, being the chemical potential difference per molecule between the dilute and condensed phases.

$G$ is dependent on the crosslink frequency of the matrix, which can be estimated by $f^2$, the square of the volume fraction of the matrix. The effective "spring constant" of the nucleus for a lamin knockout has been estimated to be on order 1 nN/$\mu$m, which dividing by a microscopic length scale of 1 $\mu$m would give about 1 kPa for the nucleus on average. Based on this one can assume that the least stiff chromatin, with a fraction of about 0.21 has a modulus of about 100 Pa and that this value scales with $f^2$.

To run the simulation, the minimal mathematical model for condensate thermodynamics within an elastic medium was implemented within a diffuse-interface formalism that permits numerical study of collective droplet nucleation, growth, and coarsening kinetics with mechanical effects. The nucleoplasmic fluid is described as an effectively ternary system composed of dCas9-ST + scFV-sfGFP-iLID (species A), TR-mCh-sspB (species B; TR=Transcriptional Regulator), and other "solvent" molecules (species C). An extended ternary regular solution free energy functional is employed to describe fluid thermodynamic phase behavior within a mechanical network:

$F = \int [\phi_A \ln \phi_A + \phi_B \ln \phi_B + \phi_C \ln \phi_C + \chi_{AB}\phi_A\phi_B + \chi_{AC}\phi_A\phi_C + \chi_{BC}\phi_B\phi_C +$

$\sum_i [\tilde{\lambda}_i(\vec{r}, R)\nabla\phi_i]^2 + \frac{5G(\vec{r})(\phi_A+\phi_A)}{6} + P_A(\vec{r})(1 - \phi_A)^2]d\vec{r}$, where $\phi_i$ is the space-dependent volume fraction of molecular population $i \in \{A, B, C\}$; $\chi_{ij}$ controls the strength of interaction between $i$ and $j$ molecules; $\tilde{\lambda}_i(\vec{r}, R)$ is the network-modified surface energy coefficient for population i (see below); $G(\vec{r})$ is the space-dependent Young's modulus of the network; and $P_A(\vec{r})$ is a field that enhances the concentration of species A at pre-seed sites. The fluid phase is taken to be incompressible such that $\phi_A + \phi_B + \phi_C$ = 1. Dynamics are given by a generalized diffusion equation,

$\frac{\partial\phi_i}{\partial t} = M_i\nabla^2 \frac{\partial F}{\partial\phi_i}$ where $i, j \in \{A, B\}$ ($C$ is eliminated via incompressibility), $M_i$ is the mobility of population i, and t is dimensionless time.

The effect of the elastic medium is incorporated into a space-dependent bulk term (that proportional to $G(\vec{r})$) which locally modulates the chemical potentials, and a space- and droplet size-dependent interfacial energy coefficient

$\tilde{\lambda}_i(\vec{r}, R) = \tilde{\lambda}_i + G(\vec{r})r_{mesh}(2 + \frac{r_{mesh}^3}{2R^3})/9$, where $\lambda_i$ is the surface energy coefficient in the absence of a network. The interfacial terms associated with the network are negligible compared to the bulk network term when $R \gtrsim 10r_{mesh}$. One can therefore neglect the effect of the network on interfacial energy (let $\tilde{\lambda}_i \approx \lambda_i$) and study the regime in which $R \gtrsim 10r_{mesh}$.

The telomere pre-seeding simulations were conducted by initializing a homogeneously mixed fluid with $\phi_A$ =

0.1, $\phi_B$ = 0.1, and $\phi_C$ = 0.8, $\chi_{AC}$ = $\chi_{BC}$ = 1, $\lambda_i$ = 0.75, and setting $G(\vec{r})$ = 0.6 within randomly positioned pre-seed regions, $P_A(\vec{r})$ = 0.005 within an annulus of radius 10 around each stiff pre-seed core, and $G(\vec{r})$ = $P_A(\vec{r})$ = 0 elsewhere. Non-zero $P_A$ values enhance the local concentration of A around pre-seed sites, analogous to the initial enhancement of dCas9-ST + scFV-sfGFP-iLID around telomeres in the experimental system. Blue light-induced heterodimerization of sspB with iLID is described as an increase in A-B interaction strength, $\chi_{AB} = \chi_{AB}^0 + (\chi_{AB}^{max} - \chi_{AB}^0)(1 - e^{-t/\tau[blue]}$ , where $\chi_{AB}^0 = -5$ and $\chi_{AB}^{max} = -9.25$ are the inactivated state and activated state interaction strengths, respectively. $\tau[blue]$ is the time constant for blue light activation, which is inversely proportional to the rate of increase in blue light intensity.

Following equilibration with pre-seed sites as described above, blue light is applied globally with intensity increasing according to the equation above for a given value of $\tau[blue]$. Droplets are more effectively localized at pre-seed sites with decreasing quench rate, as observed in experiments. The simulations demonstrate that enhanced concentration of A at pre-seed sites promotes rapid local nucleation and subsequent diffusion-limited growth. Growth proceeds by drawing in nearby A and B molecules, which creates an expanding radial zone depleted in A and B. If depletion zones of neighboring pre-seed sites overlap before droplets can nucleate between pre-seeds, then all droplets become localized at pre-seed sites. If droplets nucleate throughout the system before depletion zones overlap, then long-lived droplets also appear away from seed sites. For ideal diffusion-limited growth, the radius of the depletion zone grows as

$R_{DZ} \approx \sqrt{2DS_0 t}$ , where D is the diffusion coefficient of A and B molecules and $S_0$ is their supersaturation. The time required for overlap of neighboring depletion zones is therefore $t \; t^* \approx \dfrac{\left(\frac{d}{2}\right)^2}{2DS_0}$ , where d is the distance between pre-seed sites. Simulations can be performed with a spatially heterogeneous elastic network were conducted with blue light applied globally as described above, but with $P_A(\vec{r})$ = 0 and $G(\vec{r})$ = $G_0$[cos 4$\pi$x/$L_x$ + cos 4$\pi$y/$L_y$ + 2]/4, where $L_x$ and $L_y$ are the lengths of the simulation cell. The constant $G_0$ was increased linearly from 0 to 0.18 between t = 250 (after droplet nucleation) and t = 750. This delay in the introduction of mechanical deformation energy results in spatially uniform droplet nucleation and an initial droplet size distribution that is independent of network stiffness, consistent with our neo-Hookean model. The subsequent introduction of nonzero $G(\vec{r})$ induces a transition into the large droplet size regime discussed above, in which the dominant effect is a shifting of the bulk chemical potential according to local stiffness and preferential droplet growth in softer regions. Other were conducted as those discussed above, but with blue light applied locally inside a stiff heterochromatin-like domain, wherein $G_0$ was increased linearly from 0 to 0.18 between t = 100 (after droplet nucleation) and t = 600.

One can estimate targeted chromatin displacement. When two droplets seeded at specific genomic loci fuse, surface tension favors making the resulting large droplet spherical. This results in a force that attracts the two loci towards one another, displacing each one a distance Δx from its original position. This displacement induces deformation in the chromatin that results in an elastic restoring force pulling the loci back towards their original positions; at mechanical equilibrium, the balance between droplet surface area and loci displacement reflects the relative magnitude of the surface tension $\gamma$ and chromatin Young's modulus G. Quantitatively, when two droplets of radius R fuse to form a sphere, the resulting droplet has an equilibrium radius of $2^{1/3}R$. Due to surface tension, there is an energetic cost to elongating the droplet from the spherical configuration. This results in a force $F_{tension}$ between the telomeres, which to linear order in the elongation δ = 2R - $2^{1/3}$R - Δx, and assuming that the droplet is constrained to remain ellipsoidal, reads $F_{tension}$ = $\gamma$(2R - $2^{1/3}$R - Δx). The elastic force on a locus of size $r_{locus}$ displaced by Δx is given by the elastic Stokes' law, $F$elastic=-6$\pi$G$r_{locus}$Δx. Force balance thus yields

$$\Delta x \approx \gamma(2R - 2^{\frac{1}{3}}R)/(\gamma + 6\pi BGr_{locus}) \qquad\qquad (\text{Eq. 4})$$

Taking a telomere size of about 10 nm, a $\gamma$ of 4 x 10⁻⁷ N/m as before, a droplet size of 1 $\mu$m and a G in the range of 10-100 Pa results in a displacement Δx ≈ 10 - 100 nm.

Control experiments without sgRNA for telomeres show that BRD4 droplets appear in an apparently random manner irrespective of telomeres (Fig. 2a). Collectively, these results indicate that droplet localization during intracellular phase separation can be dynamically controlled by pre- seeding and the rate of supersaturation. The CasDrop system thus functions to localize a nucleating scaffold, thereby driving phase separation at the genomic location defined by the sgRNA.

In studies of off-target nucleation using CasDrops, droplets appear to form in regions of relatively low chromatin density. To quantify this, one can examine cells not expressing condensate-targeting guide RNA and determine where droplets form relative to the chromatin distribution prior to droplet condensation, using H2B-miRFP670 as a proxy for chromatin density. One can find that the distribution of H2B intensity in regions where droplets do form is shifted to significantly lower H2B intensity compared with the H2B distribution in the entire nucleus. Dividing these two distributions reveals that the propensity for droplet formation is a strong function of normalized H2B intensity, with droplets exhibiting a significant preference for regions of low chromatin density. Notably, the observed trend is not due merely to variations in the concentrations of CasDrop components, as similar analysis does not show biased distributions of the components. A similar tendency can be seen for droplet growth in low-density chromatin for different IDRs driven to phase separate using the optoDroplet system.

One can also examine the chromatin density after droplets have formed. Remarkably, it can be found that as the droplets grow chromatin is significantly pushed out, creating easily visualized "holes".

Chromatin exclusion is not an artifact of optogenetic scaffolding, as we see similar behavior for condensates formed with a variety of YFP-tagged IDR-containing full-length proteins overexpressed in cells (for genes CCNT1, HSF1, MLLT3, RNPS1, SART1, and TAF15, with a system comprising ORF-EYFP and H2B-miRFP670). These findings are consistent with the observation that several endogenous nuclear condensates, including nucleoli, Cajal bodies (CBs), PML bodies, nuclear speckles, and paraspeckles are also associated with particularly low-density chromatin. Thus, condensates assembled from IDRs exhibiting a broad spectrum of sequence features not only preferentially nucleate in low density chromatin regions, but upon nucleation and growth they also physically exclude chromatin.

To gain physical insight into why droplets that tend to exclude chromatin would preferentially grow in regions of low chromatin density, we mathematically model the mechanical interplay of condensates with the deformable chromatin network. We reasoned that by excluding chromatin, growing droplets would give rise to mechanical stresses; indeed, in non-biological systems, phase separated droplets are known to be strongly impacted by the presence of a surrounding elastic network. We developed a minimal model to describe droplet formation in chromatin as an expanding sphere creating a cavity in an elastic matrix. A simplified expression for the free energy cost $\Delta F$ of droplet nucleation is given by:

$$\Delta F(R) = 4\pi\gamma R^2 - 4/3\pi R^3\left(\Delta\mu \cdot c_{drop} - 5/6G\right)$$

where R is the radius of the droplet, $\gamma$ is the surface tension of the droplet, $\Delta\mu$ is the chemical potential difference between molecules in the supersaturated solution and in the droplet phase, cdrop is the saturated bulk concentration of molecules inside the droplet, and $G$ is the elastic (Young's) modulus of the deformed surrounding matrix. The first two terms reflect classical nucleation theory, while the third term reflects a mechanical energy contribution from the chromatin elasticity. Using estimates of the key parameters of surface tension, molecular interaction energy, and stiffness of euchromatin and heterochromatin, the model predicts that small droplets can nucleate in both soft and stiff regions, but that droplet growth to an optically resolvable scale will be most probable in soft, low-density chromatin. Simulations of the mechanical model using a sinusoidally-varying chromatin stiffness show clear droplet preference for minima in the stiffness landscape.

A prediction of this model is that mechanical deformation energy will prevent droplets from growing beyond a critical size in sufficiently dense heterochromatic regions, i.e., those near the high-density end of the experimentally measured droplet growth propensity. To test this prediction, one can examine major satellite repeats, dense heterochromatin regions prominent in mouse cultured cells, which are highly enriched in the heterochromatin protein HP1$\alpha$. The CasDrop system can be used to locally assemble BRD4 condensates by focusing the activating laser within the nucleus. When BRD4 droplets are induced in euchromatin regions, devoid of HP1$\alpha$-miRFP670 label, BRD4 droplets readily condense. However, when one attempts to write droplets directly onto heterochromatin, resolvable droplets tend to condense only around the periphery; this effect is not a result of IDR exclusion, since it robustly occurs even after enrichment of IDR-sspB component through sgRNA targeting. Simulations of condensate nucleation at a compact heterochromatin core demonstrate flower petal-like configurations. Interestingly, in some cases HP1$\alpha$-miRFP670 enriched heterochromatin foci exhibit apparent subregions of weak HP1$\alpha$- miRFP670 fluorescence, which may reflect some degree of interspersed euchromatin.

When one attempts to nucleate BRD4 droplets on these foci, one finds that BRD4 droplets occasionally nucleate within; as they grow, they displace HP1$\alpha$, as seen from the anti-correlated fluorescence intensities; strikingly, the growing BRD4 droplets can even "rupture" heterochromatin, abruptly spilling out into euchromatic regions. One can observe qualitatively similar heterochromatin immiscibility with TAF15 and FUS CasDrops.

Collectively, these behaviors provide strong support for the presence of mechanical stresses that build up in chromatin surrounding growing condensates. These data are thus consistent with the mechanical droplet exclusion model, although HP1$\alpha$ itself could provide additional unfavorable interactions to reinforce this mechanical immiscibility.

These findings of mechanical chromatin exclusion are surprising, given that enhancer clusters and other nuclear condensates have been hypothesized to bring specifically targeted genomic elements into closer proximity. To explore these mechanical effects, we further examined the condensates targeted to telomeres. Consistent with the findings above, BRD4 CasDrop condensates do not appear to mix with the targeted heterochromatic telomeres, which tend to localize at the droplet periphery. But the two nevertheless partially wet one another and are thus adherent. When two droplets seeded at different telomeres fuse with one another (Fig. 6a), the surface tension is sufficient to produce correlated motion between loci and pull them into close proximit. Ignoring viscous effects, telomere displacement should be given by the ratio of droplet surface tension, $\gamma$, and effective genome elasticity, $G$, i.e. $\Delta x \sim \gamma / G$; estimated values for these two parameters yield predicted displacements that are consistent with measurements. In some cases, droplets can detach from associated telomeres, which then relax back to a more distal position, consistent with droplet detachment releasing the intervening "spring-loaded" chromatin.

Thus, nuclear condensates can both sense and restructure their local genomic environment. A broad range of different IDR-containing proteins exclude chromatin, which in some cases manifests in large scale deformation of the chromatin network. These proteins display a remarkable diversity of their physical characteristics, exhibiting IDRs that range from relatively uncharged (*e.g.,* TAF15 N) to highly basic (*e.g.,* SRSF2 IDR) and mixed charge (*e.g.,* SART1); from relatively hydrophobic (*e.g.,* HSF1) to highly hydrophilic (*e.g.,* RNPS1). Thus, mechanical exclusion of chromatin appears to occur irrespective of the physicochemical properties of the protein that drives phase separation, and likely underlies the low chromatin density found within various IDR-rich nuclear bodies. These findings are consistent with the observation in a prior study that the germ plasm protein DDX4 appears to exclude chromatin. Interestingly, DDX4 condensates do not exclude single- stranded RNA and DNA, which instead strongly partition into the droplets. Such selectivity could potentially help facilitate the flow of genetic information, as the single-stranded RNA transcripts from excluded chromatin are pulled into adjacent condensates.

The tendency for nuclear condensates to exclude chromatin has dramatic consequences within the heterogeneous nuclear environment. Exclusion of chromatin from a growing droplet causes the chromatin network to be deformed. Deformation of elastic (or viscoelastic) materials gives rise to a strain energy, which represents a mechanical energy stored within the matrix. This deformation thus represents an energetic cost that is thermodynamically unfavorable, such that the elastic properties of the matrix become an important factor in the growth dynamics of the droplet. In non-living systems, it is known that this effect can strongly impact phase separation and can even give rise to uniform droplets of a size set by the matrix elasticity. The disclosed theoretical analysis and simulations show that as a result of this deformation energy, droplets will tend to favor growth in softer, lower density regions of the genome. Small droplets that do form in heterochromatin ultimately dissolve and act as IDR sources for the growing droplets within the softer euchromatin regions. We experimentally observed the striking consequences of this effect, in flower-petal-like arrangement of droplets around heterochromatin foci, or in rare cases the extrusion of droplets from within dense and mechanically stiffer heterochromatin. This effect can thus give rise to genomic rearrangements and is likely important for promoting preferential growth of transcriptional condensates in mechanically softer, low-density genomic regions associated with active gene expression.

Our results also shed light on how targeted condensates can bring distant loci together. Many IDR-rich proteins possess targeting "reader" motifs, such as the bromodomain found in BRD4, which targets this phase separation-prone protein to histones exhibiting acetylated lysine resides (Dey et al., 2003). The CasDrop system replaces such endogenous targeting motifs with programmable dCas9, enabling dissection of the biophysical consequences of IDP targeting. Once can use CasDrop to show how IDP targeting promotes localized phase separation, a process which appears closely related to the "diffusive capture" mechanism, which can amplify IDR concentration to drive localized phase separation. Surface-tension can mediate targeted droplet coalescence. The associated forces and genomic deformations can be quantified, which can bring two or more targeted loci into closer proximity. This ability to "pull in" targeted genomic loci, can be contrasted with our finding that a broad spectrum of IDR-driven condensates does the opposite to non-targeted genomic elements, i.e. "pushing them out". Combing these findings, we propose a chromatin filter model for condensate-induced genome restructuring, in which transcriptionally active condensates such as the nucleolus, and super-enhancer clusters, play bifunctional roles, serving to both filter out non-specific elements of the genome, while pulling together targeted regions to which they are bound.

Studies of the interplay between intracellular mechanics and phase separation have only just begun. But continued progress in this area will be important both in the context of mechanics of the cytoplasm, where cytoplasmic condensates interact with the actomyosin cytoskeleton, for example, and in the nucleus, where condensates interact with the polymeric matrix of the genome, and other mechanical elements. Within the nucleus, this challenge may be particularly rich, given that many nuclear condensates form on small size scales where local mechanical heterogeneities become important. The field of nuclear mechanobiology has identified a link between mechanical forces and gene expression. However, the underlying biophysical mechanisms are still largely unknown. Our findings that the dynamics of nuclear condensates are sensitive to the local mechanical environment suggest that the impact of mechanical forces on gene expression might ultimately be mediated by their effect on nuclear condensates. Future work will explore these emerging links between nuclear mechanics and phase separation, and their impact on functional changes in gene expression.

For the specific example disclosed herein, the following disclosed techniques were used.

*Cell culture.* NIH3T3, HEK293, HEK293T, and U2OS cells were cultured in growth medium consisting of Dulbecco's modified Eagle's medium (Gibco), 10% fetal bovine serum (Atlanta Biologicals), and 10 U/mL Penicillin-Streptomycin (Gibco), and incubated at 37°C and 5% $CO_2$ in a humidified incubator.

*Transient transfection.* HEK293, HEK293T, or U2OS cells were grown to approximately 70% confluency in 12-well plates before being transfected with plasmid DNA using Lipofectamine 3000 (Invitrogen) following manufacturer's protocol. Briefly, transfection reagents and DNA plasmids were diluted with OPTI-MEM (Gibco). Each well received 100 $\mu$L of transfection mixture containing a total of 1 $\mu$g DNA. The transfection mixture was removed 6-24 hours post-transfection. Cells transiently transfected were typically imaged between 24-48 hours post-transfection.

Lentiviral transduction. Lentivirus was produced by cotransfecting the transfer plasmids, pCMV-dR8.91, and pMD2.G (9:8:1, mass ratio) into HEK293T cells grown to approximately 70% confluency in 6-well plates using FuGENE HD Transfection Reagent (Promega) per manufacturer's protocol. A total of 3 $\mu$g plasmid and 9 $\mu$L of transfection reagent were delivered into each well. After 2 days, supernatant containing viral particles was harvested and filtered with 0.45 $\mu$m filter (Pall Life Sciences). Supernatant was immediately used for transduction, concentrated 10-fold using Lenti-X Concentrator (Takara), or stored at -80°C in aliquots. NIH3T3 or HEK293T cells were grown to 10-20% confluency in 12-well plates and 100-1000 $\mu$L of filtered viral supernatant was added to the cells. Media containing virus was replaced with fresh growth medium 24 hours post-infection. Cells infected were typically imaged no earlier than 72 hours after infection.

*Cell line generation.* To establish cell lines expressing multiple constructs, sequential lentiviral transduction was performed, together with fluorescence activated cell sorting (FACS) when needed. Wild-type NIH3T3 (or HEK293T) cells were transduced with lentivirus containing dCas9-ST under SFFV promoter and scFv-sfGFP-iLID. This transduced NIH3T3 cell line was then used to generate other cell lines by lentiviral transduction, to express required constructs indicated for each experiment. To increase population expressing dCas9-ST in the transduced HEK293T cell line, cells were sorted on a FACSAria Fusion flow cytometer (BD Biosciences) with gating for single-cells expressing high level of BFP and intermediate level of GFP. Polyclonal cell pool was collected, grown and recovered in growth medium. This sorted cell line was then transiently transfected with additional constructs for CasDrop/Cry2-fusion experiment.

*Constructs.* FUSN-mCh-sspB was first generated by inserting FUSN (1-214), mCherry, and sspB coding sequence into a pHR-based vector(Shin et al., 2017). For other TR-mCh-sspB constructs, the FUSN in FUSN-mCh-sspB was swapped out for the DNA sequence encoding BRD4ΔN (462-1362), TAF15N (1-208). scFv-sfGFP-iLID was generated by adding iLID (Addgene 60413) between GB1 and NLS in scFv-GCN4-GFP (Addgene 60906). A promoter for dCas9-ST (Addgene 60910) was modified from dSV40 to SFFV to enhance expression. Fragments of mCherry, sspB (Addgene 60415), miRFP670 (Addgene 79987), TRF1 (Addgene 64164) and HP1$\alpha$ (Addgene 17652) were amplified by PCR. All Cry2 fragments used here are identical to the one described previously(Shin et al., 2017) except for a fluorescent reporter swapped from mCh to miRFP670. For EYFP constructs, FM5-EYFP (kind gift from Marc Diamond lab, UT Southwestern) was digested with NheI and open-reading frames were subcloned onto 5' end using In-Fusion Cloning Kit (Takara). To create FMS-ORF-mCherry-Cry2 constructs, FM5-EYFP was digested with NheI and AscI and DNA backbone lacking EYFP was gel purified using Qiagen Gel Extraction Kit. mCherry-Cry2 was PCRed from pHr-mCherry-Cry2, digested with NheI and AscI, gel purified, and ligated into FM5 backbone using Quick Ligase (NEB). FM5-mCherry-Cry2 was then digested with NheI and open reading frames were subcloned onto 5' end using In-Fusion. All open reading frames were PCRed from recombinant DNA vectors obtained from AddGene: CCNT1 (14607), HSF1 (32538), MLLT3 (49428), SART1 (38087), TAF15 (84896), BMI1 (69796), SRSF2 (84020), PRPF6 (51740), with the exception of RNPS1, which was synthesized by GenScript. For sgRNAs targeting telomeres and major satellite repeats (sgTel: TTAGGGTTAGGGTTAGGGTTA [SEQ ID NO.: 1]and sgMaj: CAAGAAAACTGAAAATCA [SEQ ID NO.: 2]), pLV-sgCDKNIB (Addgene 60905) was first digested with BstXI and XhoI followed by gel electrophoresis and extraction. Then, PCR fragments for sgRNAs were generated using a sequence-specific forward primer (5'-CCCTTGGAGAACCACCTTGTTGGNxGTTTAAGAGCTATGCTGGAAACAGCA-3' [SEQ ID NO.: 3], where GNx is the base pairing sequence) and a common reverse primer (5'-GATCCTAGTACTCGAGAAAAAAAGCACCG-3' [SEQ ID NO.: 4]). All fragments were ligated and assembled into the final vectors using In-Fusion Cloning Kit (Takara).

*Immunocytochemistry.* HEK293 cells expressing H2B-miRFP670 were fixed using 3.5% PFA (Electron Microscopy Services) in PBS for 15 minutes. Cells were washed twice with PBS and permeabilized with 0.25% Triton-X in PBS for 20 minutes. Non-specific epitopes were blocked for one hour using blocking buffer (PBS, 0.1% Triton-X, 10% normal goat serum from Vector Laboratories). Primary immunostaining was performed with the following antibodies overnight at 4°C in blocking buffer: PML (Mouse, AbCam ab11826, 1 to 50), Coilin (Rabbit, Santa Cruz sc32860, 1 to 100), TDP43 (Rabbit, ProteinTech 10782-2-AP, 1 to 100), SMN1 (Mouse, Santa Cruz sc-32313, 1 to 100), SC35 (Mouse, AbCam ab11826, 1 to 1000), and FBL (Mouse, AbCam ab4566, 1 to 40). Cells were then washed 3X with 0.1% Triton-X in PBS. Secondary immunostaining was performed with the following antibodies from Invitrogen in blocking buffer at room temperature for 90 minutes: AlexaFluor 546 goat anti-rabbit (A11010, 1 to 400), AlexaFluor 546 goat anti-mouse (A11030, 1 to 400). Cells were washed 3X with 0.1% Triton-X in PBS. DNA was visualized with 2 $\mu$g/mL Hoechst dye

(ThermoScientific), staining for 15 minutes in PBS. Finally, Hoechst was removed and replaced with PBS prior to imaging. Controls without primary antibodies were performed to ensure specificity of primary stain.

*Microscopy.* All images were taken using 60X immersion objective (NA 1.4) on a Nikon A1 laser scanning confocal microscope. An imaging chamber is maintained at 37°C and 5% CO2. For live cell imaging, cells are plated on the fibronectin (Sigma-Aldrich) coated 35-mm glass-bottom dishes (MatTek) and grown typically overnight. For global activation, cells are usually imaged with a 488-nm laser but when the blue light intensity needs to be reduced due to high sensitivity of the optogenetic proteins (iLID and Cry2), a 440-nm laser is used in conjunction with a dichroic filter for the 488-nm laser. This allows for attenuation of the blue laser intensity at the specimen plane below 0.1 $\mu$W. For local activation, a region of interest (ROI) is defined to guide area to be scanned with blue lasers. Fluorescence recovery after photobleaching (FRAP) is performed similarly using ROI.

*Image analysis.* All data analysis on images was performed using custom-built MATLAB scripts. Briefly, for telomere tracking, raw images are first Gaussian filtered to reduce noise and then peaks corresponding to telomeres are detected based on their peak intensity. Trajectories are generated from a series of detected coordinates based on proximity. To identify and track the boundary of either droplets or heterochromatin, segmented binary images are obtained using the edge detection routine in MATLAB. Analyzed results are manually inspected to check validity.

## Claims

1. A high-throughput method for mapping or screening intracellular interactions in vitro, comprising the steps of:

    a. providing a plurality of cells, each cell expressing a phase separation or aggregation system capable of being controlled by at least one wavelength of light, the phase separation or aggregation system comprising (i) a target protein and (ii) a fluorescent protein or an attached fluorophore;
    b. placing at least one of the plurality of cells in a well at a first temperature;
    c. introducing at least one chemical or biological agent to the well at a first concentration;
    d. irradiating the well with the at least one wavelength of light, in a constant or pulsed fashion, and allowing the phase separation or aggregation system to form condensates;
    e. irradiating the at least one of the plurality of cells with an additional wavelength of light to cause the fluorescent protein or an attached fluorophore to fluoresce;
    f. quantifying phase separation or aggregation based on an amount of fluorescence within a first region and a second region of the plurality of cells, the first region containing a condensate and the second region not containing a condensate, to map or screen intracellular interactions.

2. The method according to claim 1, further comprising fixing and staining the at least one of the plurality of cells.

3. The method according to claim 1, wherein the phase separation or aggregation system is an optoDroplet, CasDrop, PixELL or Corelet system.

4. The method according to claim 1, wherein the cell is configured to express a phase separation or aggregation system comprising:

    a first construct comprising enzymatically dead Cas9 fused or attached to two or more repeating sequences, each repeating sequence including a receptor protein sensitive to at least one wavelength of light, and
    a second construct comprising a cognate partner of the light-sensitive receptor protein fused to at least one fluorescent protein and at least one gene regulatory protein having a full length or truncated low complexity or intrinsically-disordered protein region, or other folded proteins known to promote at least one of self-interactions, a network of heterotypic (non-self) interactions, or phase separation.

5. The method according to claim 1, wherein the well is irradiated with the at least one wavelength of light, in continuous or pulsed fashion, before at least one chemical or biological agent is introduced to the well.

6. The method according to claim 1, wherein at least one chemical or biological agent is a compound from a small molecule library.

7. The method according to claim 1, wherein at least one chemical or biological agent is a component of a genetic knockout or knockdown screening system, preferably wherein the genetic knockout or knockdown screening system is selected from the group consisting of TALEN, shRNA, siRNA and CRISPR-KO.

8. The method according to claim 1, further comprising capturing an image of the at least one of the plurality of cells while the fluorescent protein or attached fluorophore is fluorescing.

9. The method according to claim 8, further comprising utilizing image processing software to identify at least one potential first region and at least one potential second regions, preferably further comprising measuring pixel intensity within at least one of the potential first and second regions.

10. The method according to claim 8, wherein

quantifying phase separation or aggregation comprises determining a standard deviation of the measured pixel intensity; or
quantifying phase separation comprises determining a threshold in changes in image parameters that allow determination of percentage of cells in a given well that phase separate; or
toxicity associated with assay conditions is determined by examining a metric comprised of the number of viable cells detected within each well, by image analysis algorithms; or
the image is sent to a trained machine learning algorithm, preferably wherein the trained machine learning algorithm is trained to estimate a first and a second concentrations based on the image.

11. The method according to claim 1, wherein steps d-f are repeated under different conditions selected from the group consisting of different light conditions, and different temperature conditions, preferably wherein a time-dependent transition into an irreversible aggregation state is monitored by changing the time that the cells are subject to constant or pulsed activating light.

12. The method according to claim 1, further comprising generating a full phase diagram utilizing the quantified phase separation or aggregation.

13. The method according to claim 1, further comprising calculation a difference between the quantified phase separation or aggregation and a baseline phase separation or aggregation, preferably further comprising signaling to a user when the difference exceeds a predetermined threshold.

14. The method according to claim 1, further comprising identifying a false-positive hit by using an optogenetic system complementary to the phase separation or aggregation system capable of being controlled by at least one wavelength of light.

15. The high-throughput method according to claim 1, further comprising:
g. determining the phase boundary for the phase separation or aggregation system, including the full binodal and spinodal phase boundaries, and monitoring the location of these boundaries, in full or in part, under the action of at least one chemical or biological agent.

**Patentansprüche**

1. Hochdurchsatzverfahren zur Kartierung oder zum Screening intrazellulärer Wechselwirkungen in vitro, umfassend die Schritte:

a. Bereitstellen einer Vielzahl von Zellen, wobei jede Zelle ein Phasentrenn- oder Aggregationssystem exprimiert, das durch mindestens eine Lichtwellenlänge gesteuert werden kann, wobei das Phasentrenn- oder Aggregationssystem (i) ein Zielprotein und (ii) ein fluoreszierendes Protein oder ein angehängtes Fluorophor umfasst;
b. Platzieren mindestens einer der mehreren Zellen in einer Vertiefung bei einer ersten Temperatur;
c. Einbringen mindestens eines chemischen oder biologischen Wirkstoffs in einer ersten Konzentration in die Vertiefung;
d. Bestrahlen der Vertiefung mit der mindestens eine Lichtwellenlänge in einer konstanten oder gepulsten Weise und Ermöglichen, dass das Phasentrenn- oder Aggregationssystem Kondensate bildet;
e. Bestrahlen der mindestens einen der mehreren Zellen mit einer zusätzlichen Lichtwellenlänge, um zu bewirken, dass das fluoreszierende Protein oder ein angehängtes Fluorophor fluoresziert;
f. Quantifizieren der Phasentrennung oder -aggregation auf Grundlage einer Fluoreszenzmenge innerhalb einer ersten Region und einer zweiten Region der mehreren Zellen, wobei die erste Region ein Kondensat enthält und die zweite Region kein Kondensat enthält, um intrazelluläre Wechselwirkungen abzubilden oder zu screenen.

2. Verfahren nach Anspruch 1, ferner umfassend Fixieren und Färben der mindestens einen der mehreren Zellen.

3. Verfahren nach Anspruch 1, wobei das Phasentrenn- oder Aggregationssystem ein Opto-Droplet-, CasDrop-, PixELL- oder Corelet-System ist.

4. Verfahren nach Anspruch 1, wobei die Zelle dazu konfiguriert ist, ein Phasentrennungs- oder Aggregationssystem zu exprimieren, umfassend:

ein erstes Konstrukt, das enzymatisch abgestorbenes Cas9 umfasst, das an zwei oder mehr sich wiederholende Sequenzen fusioniert oder angehängt ist, wobei jede sich wiederholende Sequenz ein Rezeptorprotein enthält, das für mindestens eine Lichtwellenlänge empfindlich ist, und
ein zweites Konstrukt, das einen kognitiven Partner des lichtempfindlichen Rezeptorproteins, das an mindestens ein fluoreszierendes Protein fusioniert ist, und mindestens ein genregulatorisches Protein mit einer vollständigen oder verkürzten Region geringer Komplexität oder einer intrinsisch gestörten Proteinregion oder andere gefaltete Proteine umfasst, von denen bekannt ist, dass sie mindestens eine von Selbstwechselwirkungen, ein Netzwerk heterotypischer (Nicht-Selbst-)Wechselwirkungen oder Phasentrennung fördern

5. Verfahren nach Anspruch 1, wobei die Vertiefung kontinuierlich oder gepulst mit der mindestens einen Lichtwellenlänge bestrahlt wird, bevor mindestens ein chemischer oder biologischer Wirkstoff in die Vertiefung eingeführt wird.

6. Verfahren nach Anspruch 1, wobei mindestens ein chemischer oder biologischer Wirkstoff eine Verbindung aus einer kleinen Molekülbibliothek ist.

7. Verfahren nach Anspruch 1, wobei mindestens ein chemischer oder biologischer Wirkstoff eine Komponente eines genetischen Knockout- oder Knockdown-Screeningsystems ist, wobei das genetische Knockout- oder Knockdown-Screeningsystem vorzugsweise aus der Gruppe ausgewählt ist, die aus talen, shRNA, siRNA und CRISPR-KO besteht.

8. Verfahren nach Anspruch 1, ferner umfassend das Aufnehmen eines Bildes der mindestens einen der mehreren Zellen, während das fluoreszierende Protein oder der angehängte Fluorophor fluoresziert.

9. Verfahren nach Anspruch 8, ferner umfassend Verwenden von Bildverarbeitungssoftware, um mindestens eine potenzielle erste Region und mindestens eine potenzielle zweite Region zu identifizieren, vorzugsweise ferner umfassend Messen der Pixelintensität innerhalb mindestens einer der potenziellen ersten und zweiten Regionen.

10. Verfahren nach Anspruch 8, wobei

quantifizieren der Phasentrennung oder -aggregation das Bestimmen einer Standardabweichung der gemessenen Pixelintensität umfasst; oder
quantifizieren der Phasentrennung das Bestimmen eines Schwellenwerts für Änderungen der Bildparameter umfasst, die die Bestimmung des Prozentsatzes von Zellen in einer gegebenen Vertiefung, die sich in der Phase trennen, ermöglichen; oder
die mit den Assay-Bedingungen assoziierte Toxizität durch Untersuchung einer Metrik bestimmt wird, die aus der Anzahl der in jeder Vertiefung detektierten lebensfähigen Zellen besteht, und zwar durch Bildanalysealgorithmen; oder
das Bild an einen trainierten maschinellen Lernalgorithmus gesendet wird, wobei der trainierte maschinelle Lernalgorithmus vorzugsweise darauf trainiert ist, eine erste und eine zweite Konzentration auf der Grundlage des Bildes zu schätzen.

11. Verfahren nach Anspruch 1, wobei die Schritte d-f unter verschiedenen Bedingungen wiederholt werden, die aus der Gruppe ausgewählt werden, die aus verschiedenen Lichtbedingungen und verschiedenen Temperaturbedingungen besteht, wobei vorzugsweise ein zeitabhängiger Übergang in einen irreversiblen Aggregationszustand überwacht wird, indem die Zeit verändert wird, in der die Zellen konstantem oder gepulstem aktivierendem Licht ausgesetzt sind.

12. Verfahren nach Anspruch 1, ferner umfassend Erzeugen eines vollständigen Phasendiagramms unter Verwendung der quantifizierten Phasentrennung oder -aggregation.

13. Verfahren nach Anspruch 1, ferner umfassend Berechnen einer Differenz zwischen der quantifizierten Phasentrennung oder -aggregation und einer Basislinienphasentrennung oder - aggregation, vorzugsweise ferner umfassend das Signalisieren an einen Benutzer, wenn die Differenz einen vorbestimmten Schwellenwert überschreitet.

14. Verfahren nach Anspruch 1, ferner umfassend Identifizieren eines falsch-positiven Treffers unter Verwendung eines optogenetischen Systems, das komplementär zu dem Phasentrenn- oder Aggregationssystem ist, das durch mindestens eine Lichtwellenlänge gesteuert werden kann.

15. Das Hochdurchsatzverfahren nach Anspruch 1, das ferner umfasst:
g. Bestimmen der Phasengrenze für das Phasentrenn- oder Aggregationssystem, einschließlich der vollständigen binodalen und spinodalen Phasengrenzen, und die vollständige oder teilweise Überwachung der Lage dieser Grenzen unter Einwirkung mindestens eines chemischen oder biologischen Arbeitsstoffs.

## Revendications

1. Procédé à haut rendement pour mapper ou cribler des interactions intracellulaires *in vitro,* comprenant les étapes consistant à :

   a. fournir une pluralité de cellules, chaque cellule exprimant un système de séparation de phase ou d'agrégation capable d'être contrôlé par au moins une longueur d'onde de lumière, le système de séparation de phase ou d'agrégation comprenant (i) une protéine cible et (ii) une protéine fluorescente ou un fluorophore lié ;
   b. placer au moins une cellule de la pluralité de cellules dans un puits à une première température ;
   c. introduire au moins un agent chimique ou biologique dans le puits à une première concentration ;
   d. irradier le puits avec l'au moins une longueur d'onde de lumière, de manière constante ou pulsée, et permettre au système de séparation de phase ou d'agrégation de former des condensats ;
   e. irradier l'au moins une cellule de la pluralité de cellules avec une longueur d'onde de lumière supplémentaire pour amener la protéine fluorescente ou un fluorophore lié à devenir fluorescent ;
   f. quantifier la séparation de phase ou l'agrégation en fonction d'une quantité de fluorescence dans une première région et une seconde région de la pluralité de cellules, la première région contenant un condensat et la seconde région ne contenant pas de condensats, pour mapper ou cribler les interactions intracellulaires.

2. Procédé selon la revendication 1, comprenant en outre la fixation et la coloration de l'au moins une cellule de la pluralité de cellules.

3. Procédé selon la revendication 1, dans lequel le système de séparation de phase ou d'agrégation est un système optoDroplet, CasDrop, PixELL ou Corelet.

4. Procédé selon la revendication 1, dans lequel la cellule est configurée pour exprimer un système de séparation de phase ou d'agrégation comprenant :

   une première construction comprenant Cas9 enzymatiquement mort fusionné ou lié à deux ou plusieurs séquences répétitives, chaque séquence répétitive comprenant une protéine réceptrice sensible à au moins une longueur d'onde de lumière, et
   une seconde construction comprenant un associé apparenté de la protéine réceptrice sensible à la lumière fusionnée à au moins une protéine fluorescente et au moins une protéine de régulation de gène ayant une région protéique de longueur complète ou tronquée de faible complexité ou intrinsèquement désordonnée, ou d'autres protéines repliées connues pour favoriser au moins l'un parmi des auto-interactions, un réseau de (non auto) interactions hétérotypiques, ou une séparation de phase.

5. Procédé selon la revendication 1, dans lequel le puits est irradié avec ladite au moins une longueur d'onde de lumière, de manière continue ou pulsée, avant qu'au moins un agent chimique ou biologique ne soit introduit dans le puits.

6. Procédé selon la revendication 1, dans lequel au moins un agent chimique ou biologique est un composé provenant d'une banque de petites molécules.

**7.** Procédé selon la revendication 1, dans lequel au moins un agent chimique ou biologique est un composant d'un système de knock-out génétique ou de criblage de knock-down, de préférence dans lequel le système de knock-out génétique ou de criblage de knock-down est choisi dans le groupe constitué de TALEN, shRNA, siRNA et CRISPR-KO.

**8.** Procédé selon la revendication 1, comprenant en outre la capture d'une image de l'au moins une cellule de la pluralité de cellules pendant que la protéine fluorescente ou le fluorophore lié est fluorescent.

**9.** Procédé selon la revendication 8, comprenant en outre l'utilisation d'un logiciel de traitement d'image pour identifier au moins une première région potentielle et au moins une seconde région potentielle, comprenant de préférence en outre la mesure de l'intensité de pixel dans au moins l'une des première et seconde régions potentielles.

**10.** Procédé selon la revendication 8, dans lequel

la quantification de la séparation de phase ou de l'agrégation comprend la détermination d'un écart type de l'intensité de pixel mesurée ; ou
la quantification de la séparation de phase comprend la détermination d'un seuil de changements dans les paramètres d'image qui permettent la détermination du pourcentage de cellules dans un puits donné qui se séparent en phase ; ou
la toxicité associée aux conditions de test est déterminée en examinant une métrique composée du nombre de cellules viables détectées dans chaque puits, à l'aide d'algorithmes d'analyse d'image ; ou
l'image est envoyée à un algorithme d'apprentissage automatique formé, de préférence dans lequel l'algorithme d'apprentissage automatique formé est formé pour estimer une première et une seconde concentrations en fonction de l'image.

**11.** Procédé selon la revendication 1, dans lequel les étapes d à f sont répétées dans différentes conditions choisies dans le groupe constitué de différentes conditions de lumière et de différentes conditions de température, de préférence dans lequel une transition dépendante du temps vers un état d'agrégation irréversible est surveillée en modifiant le temps pendant lequel les cellules sont soumises à une lumière d'activation constante ou pulsée.

**12.** Procédé selon la revendication 1, comprenant en outre la génération d'un diagramme de phase complet utilisant la séparation de phase ou l'agrégation quantifiée.

**13.** Procédé selon la revendication 1, comprenant en outre le calcul d'une différence entre la séparation de phase ou l'agrégation quantifiée et une séparation de phase ou une agrégation de référence, comprenant de préférence en outre la signalisation à un utilisateur du moment où la différence dépasse un seuil prédéterminé.

**14.** Procédé selon la revendication 1, comprenant en outre l'identification d'un faux-positif atteint en utilisant un système optogénétique complémentaire au système de séparation de phase ou d'agrégation capable d'être contrôlé par au moins une longueur d'onde de lumière.

**15.** Procédé à haut rendement selon la revendication 1, comprenant en outre les étapes consistant à :
g. déterminer la limite de phase pour le système de séparation de phase ou d'agrégation, comprenant les limites de phase binodale et spinodale complètes, et surveiller l'emplacement de ces limites, dans leur ensemble ou en partie, sous l'action d'au moins un agent chimique ou biologique.

100

110 — **Provide Cells**

120 — **Add Cells To Wells**

130 — **Add Agent(s)**

140 — **Form Condensates**

150 — **Fluoresce**

160 — **Detect**

175

170 — **Quantify**

180 — **Build Phase Diagram**

190 — **Compare**

*FIG. 1*

FIG. 2A

FIG. 2B

**FIG. 3**

FIG. 4A

FIG. 4B    FIG. 4C    FIG. 4D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62734063
- US 20170355977 A
- US 2019014666 W
- US 20180251497 A

### Non-patent literature cited in the description

- **DINE et al.** Protein Phase Separation Provides Long-Term Memory of Transient Spatial Stimuli. *Cell Systems,* 27 June 2018, vol. 6, 655-663